(19) Europäisches Patentamt
European Patent Office
Office européen des brevets

(11) Publication number: **0 103 632 B1**

(12) **EUROPEAN PATENT SPECIFICATION**

(45) Date of publication of patent specification: **19.12.90**

(51) Int. Cl.$^5$: **C 12 P 21/00, C 12 P 19/34, C 12 N 15/00**

(21) Application number: **83901452.9**

(22) Date of filing: **08.03.83**

(86) International application number: **PCT/US83/00310**

(87) International publication number: **WO 83/03259 29.09.83 Gazette 83/23**

# (54) METHOD FOR INTRODUCING CLONED, AMPLIFIABLE GENES INTO EUCARYOTIC CELLS AND FOR PRODUCING PROTEINACEOUS PRODUCTS.

(30) Priority: **15.03.82 US 358206**

(43) Date of publication of application: **28.03.84 Bulletin 84/13**

(45) Publication of the grant of the patent: **19.12.90 Bulletin 90/51**

(84) Designated Contracting States: **AT BE CH DE FR GB LI LU NL SE**

(56) References cited:

| | |
|---|---|
| WO-A-81/02425 | US-A-4 357 421 |
| WO-A-81/02426 | US-A-4 363 877 |
| WO-A-82/00158 | US-A-4 364 935 |
| US-A-4 262 090 | US-A-4 374 927 |

**CONFERENCE ON GENE AMPLIFICATION, 1981, & PROCEEDINGS, published in 1982, edited by R.T. Schimke, Cold Spring Harbor Lab., N.Y., pages 251-255; J.M. ROBERTS et al.: "Amplification and correction of transformed genes"**

**The file contains technical information submitted after the application was filed and not included in this specification**

(73) Proprietor: **THE TRUSTEES OF COLUMBIA UNIVERSITY IN THE CITY OF NEW YORK**
**West 116th Street and Broadway**
**New York, New York 10027 (US)**

(72) Inventor: **AXEL, Richard**
**410 Riverside Drive**
**New York, NY 10025 (US)**
Inventor: **ROBERTS, James Michael**
**134 Haven Avenue**
**New York, NY 10032 (US)**

(74) Representative: **Henkel, Feiler, Hänzel & Partner**
**Möhlstrasse 37**
**D-8000 München 80 (DE)**

(56) References cited:
**CELL, vol. 29, no. 1, May 1982, pages 109-119, MIT Press; J.M. ROBERTS et al.: "Gene amplification and gene correction in somatic cells"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
PROCEEDINGS OF THE NATIONAL ACADEMY OF SCIENCES OF THE USA, vol. 79, no. 6, March 1982, pages 1815-1819, Washington, US; J.K. CHRISTMAN et al.: "Amplification of expression of hepatitis B surface antigen in 3T3 cells cotransfected with a dominant-acting gene and cloned viral DNA"

Proceedings of the National Academy of Sciences USA, Vol. 77, issued 1980, "Transformation of Mammalion cells with an amplifiable Dominant-acting gene", M Wigler, see pp 3567-3570.

Science, Volume 208, issued 1980, "Insertion of a New Gene of Viral Origin into the Bone Marrow Cells of Mice", K.E. Mercola, see pp 1033-1035.

Cell, vol. 25, issued 1981, "Analysis of Transcripotional Regulatory Signals ofthe HSV Thymidine Kinase Gene: Identification of an Upstream Control Region", S.L. Mc Knight, see pp 385-398.

Journal of Biological Chemistry, issued 1979, "Gene Amplification Causes Overproduction of the First Three Enzymes of UMP Synthesis in N-(Phosphonacetyl)-L-aspartate-resistant Hamster Cells", G.M. Wahl, see pp. 8678-8689

Journal of Biological Chemistry, issued 1978, "Selective Multiplication of Dihydrofolate Reductase Genes in Methotrexate-resistant Variants of Cultured Murine Cells", F.W. Alt, see pp 1357-1370.

Nature, vol. 294, issued 1981, "Glucocorticoids regulate expression of dihydrofolate reductase cDNA in mouse mammary tumour virus chimaeric plasmids", F. Lee, see pp 228-232.

**Description**

Field of the invention

This invention relates to the introduction and expression in eucaryotic cells of deoxyribonucleic acid molecules (DNA) which include genes coding for proteinaceous products. The invention may be used to modify the genetic make-up of cells derived from or associated with organisms classified under the SuperKingdom *Eucaryotes*, including organisms of the Plant and Animal Kingdoms. More particularly, the invention concerns improved methods of DNA-mediated gene transfer for introduction of cloned, amplifiable genes into eucaryotic cells and for production of products encoded thereby.

Background of the invention

The use of cotransformation to introduce foreign DNA into eucaryotic cells has been described. *PCT International Publication* Numbers WO81/02425 and WO81/02426, published 3 September 1981, and scientific articles identified therein. Gene amplification as a means for obtaining enhanced gene expression has also been described. Alt, F. W., et al., (1978). J. Biol. Chem. *253*, 1357—1370, and Wahl, G. M., et al., (1979). J. Biol. Chem. *254*, 8678—8689. Eucaryotic gene promoters are known. McKnight, S. L. et al., (1981). Cell *25*, 385—398. Finally, cloned amplifiable genes have been described. Lee, D. E., et al., (1981). Nature *294*, 228—232.

Known, cloned amplifiable genes whose amplification can be selected for include those whose product either (a) directly or indirectly interacts with an inhibitor of cell growth so as to render the inhibitor ineffective, or (b) is necessary for cell survival and can be inhibited by exogenously supplied substances. In both instances, the nature of the amplification process is such that increasing amounts of gene product must be produced in the presence of increasing amounts of inhibitor in order for cells to survive.

Gene products of type (b) include dihydrofolate reductase (DHFR) and aspartate transcarbamoylase (ATCase), which are amplified in cells resistant to high concentrations of methotrexate and PALA, respectively. The ATCase gene has been cloned. However, it has not been transformed into animal cells and shown to amplify. The intact DHFR gene has not been cloned. Expressing cDNAs of DHFR have been constructed and cloned, but have not been shown to amplify.

Gene products of type (a) include the cloned, bacterial neomycin resistance gene whose expression in eucaryotes has been demonstrated. However, amplification of this gene has not been demonstrated.

The present invention discloses an unexpected, alternative route to amplification. Specific mutations can be introduced into genes coding for selectable or identifiable biochemical markers so as to render the genes functionally deficient. Following transformation, rare variant cell subclones can be identified which greatly amplify the expression of the mutant gene so that expression occurs at levels characteristic of the normal gene.

Specifically, a system involving gene transfer has been developed to study the nature of genetic rearrangements which are responsible for activation of mutant genes in mammalian cells. Cotransformation has been used to introduce a single copy of a plasmid containing an intact adenine phosphoribosyltransferase (aprt) gene along with a truncated thymidine kinase (tk) gene lacking its promoter sequence, into $aprt^- tk^-$ mouse cells. Cells containing this plasmid transform to the $aprt^+$ phenotype but remain $tk^-$. Rare variants which mutate to the $tk^+$ phenotype were considered likely to reveal some form of DNA rearrangement. All $tk^+$ variants analyzed result from dramatic amplification of the promoterless tk gene along with significant lengths of flanking sequences. No other source of rearrangements are observed in over 40 $tk^+$ mutants examined.

One striking feature of these amplified $aprt^+ tk^+$ clones is the rate at which $aprt^- tk^+$ variants appear. Examination of the structure of the amplified DNA indicates that all amplified units within a given $aprt^-$ cell bear an identical mutation. These data suggest that a correction procession is occurring at high frequency which may be designed to maintain sequence homogeneity within repeated genetic elements.

Summary of the invention

According to the invention, multiple copies of foreign DNA I are introduced into suitable eucaryotic cells by cotransforming the cells with DNA I and with a deoxyribonucleic acid molecule comprising a foreign DNA II and a foreign DNA III said DNA II and DNA III carried on the same deoxyribonucleic acid molecule, said foreign DNA II includes a functionally deficient, amplifiable gene coding for a first selectable or identifiable trait and said foreign DNA III includes a functional, amplifiable gene coding for a different second selectable or identifiable trait and whose primary promoter has been removed, said foreign DNA III including a functional amplifiable gene which is derived from a gene coding for production of adenine phosphoribosyltransferase, said foreign DNA I including a functional gene coding for a desired proteinaceous product. Cotransformation is carried out under suitable conditions permitting expression of the second trait encoded by DNA III, but not of the first trait encoded by DNA II. Cotransformed eucaryotic cells expressing of the trait the encoded by DNA II. The cloned, cotransformed cells are selected or identified on the basis of expression of the trait encoded by DNA II and recovered. The recovered cells contain multiple copies of DNA I and can be used to produce messenger RNA transcript or proteinaceous product.

Transformation vectors which can be used to introduce foreign DNA into suitable eucaryotic cells are also disclosed.

Brief description of the drawing

Fig. 1. Figure 1 shows the restriction map of a recombinant plasmid, pdLAT-3, which contains Tk and Aprt gene sequences. More specifically, plasmid pdLAT-3 contains a 2.8 kb Bgl II/Bam HI fragment of the Herpes Simplex Virus genome inserted into the Bam HI site of the vector pBR322. This fragment includes the entire coding sequences of the thymidine kinase gene (■) as well as 1.6 kb of 3′ flanking DNA (▨), but lacks the promoter and 5′ flanking DNA sequences. The initiator AUG is located 50 nucleotides 3′ to the Bgl II site. Additional in-phase AUG codons are noted. This plasmid also contains a 4.3 kb Hind III/Bgl II fragment of hamster DNA which contains the entire hamster aprt gene as well as approximately 700 bp of 5′ flanking DNA (▭). A 2.4 kb Bgl II insert of bacteriophage lambda DNA (λ), originating from positions 36489 to 38935 of the phage genome, separates the hamster and herpes virus DNA sequences. The tk and aprt genes are inserted with opposite polarities.

According to the invention, multiple copies of foreign DNA I are introduced into suitable eucaryotic cells by cotransforming the cells with DNA I and with a deoxyribonucleic acid molecule comprising a foreign DNA II and a foreign DNA III said DNA II and DNA III carried on the same deoxyribonucleic acid molecule, said foreign DNA II includes a functionally deficient, amplifiable gene coding for a first selectable or identifiable trait and said foreign DNA III includes a functional, amplifiable gene coding for a different second selectable or identifiable trait and whose primary promoter has been removed, said foreign DNA III including a functional amplifiable gene which is derived from a gene coding for production of adenine phosphoribosyltransferase, said foreign DNA I including a functional gene coding for a desired proteinaceous product. Cotransformation is carried out under suitable conditions permitting expression of the second trait encoded by DNA III, but not of the first trait encoded by DNA II. Cotransformed eucaryotic cells expressing of the trait encoded by DNA II. The cloned, cotransformed cells are selected or identified on the basis of expression of the trait encoded by DNA II and recovered. The recovered cells contain multiple copies of DNA I and can be used to produce messenger RNA transcript or proteinaceous product.

In the invention, doubly mutant cells which do not express either the trait associated with the gene on DNA II or the trait associated with the gene on DNA III may be employed. Preferably, one or both traits are associated with dominant acting genes so that the use of mutant cell lines is not necessary. Thus, for example, use of a gene associated with neomycin resistance confers upon recipient cells a trait not expressed by normal cells so that use of mutant cell lines is not required.

Generally, a suitable eucaryotic cell useful in the practice of the invention is a cell which does not produce the proteinaceous product encoded by the amplifiable gene carried on DNA I and which does not express the selectable or identifiable trait encoded by the functionally deficient, amplifiable gene carried on DNA II. However, the invention may be used to obtain enhanced production of a product which is expressed by the cell, but in amounts which are substantially less than the amounts obtained if multiple copies of DNA I are introduced into the cell. Similarly, the invention may be used to obtain enhanced expression of a gene associated with a selectable or identifiable trait where the gene, but noy the associated selectable or identifiable trait, is expressed by the cell.

Various types of eucaryotic cells may be used in practicing the invention, including cells of animal or plant origin and yeast cells. Mammalian cells are considered particularly suitable for the production of certain products such as human growth hormone or human serum albumen.

Methods of recovering cotransformed cells, of cloning and subcloning such cells and of selecting or identifying cotransformed cells on the basis of phenotypic or genotypic traits, including appropriate conditions, are known to those skilled in the art.

Preferably, DNA I and the molecule carrying DNA II and DNA III are cloned, carrier-free genes and are two separate molecules treated with calcium phosphate prior to cotransformation. The ratio of cloned DNA I to cloned molecule, e.g., plasmid, carrying DNA II and DNA III may vary widely. Preferably, the ratio is in the range from about 650:1 to 25:1.

Foreign DNA I includes a gene coding for production of a protein or protein-containing product. Examples are human and animal growth hormone, insulin, an interferon, a clotting factor, an influenza virus antigen, a hepatitus virus antigen, human serum albumen, an antibody or an enzyme.

The functionally deficient, amplifiable gene on DNA II and the functional, amplifiable gene on DNA III may vary widely. Suitable examples of DNA II have already been mentioned. Suitable examples of DNA III are in general selected from the same group, except that the genes on DNA III are not characterized by a functional deficiency.

In other respects, the method involving use of DNAs I, II and III will be the same as that involving DNAs I and II. Any variations or modifications employed would be apparent to one skilled in the art. As with the process involving use of DNAs I and II, the process involving use of DNAs I, II and III can be employed to produce and recover messenger RNA transcripts of the DNA and of the proteinaceous products encoded thereby.

In practicing the invention, deoxyribonucleic acid molecules are used as transformation vectors. Such molecules include those which carry two foreign DNAs, one of which is a foreign DNA II, and additional DNA derived from a noneucaryotic source, e.g.,. a bacterial plasmid such as pBR322. In the preferred

embodiment of the invention, the molecules are circular and include foreign DNA III, foreign DNA II and DNA derived from a noneucaryotic source, e.g., a bacterial plasmid or bacteriophage. An example of such a vector is shown in Fig. 1.

One application of the invention involves its use to introduce into murine Ltk⁻ aprt⁻ cells multiple copies of a cloned, carrier-free gene for human growth hormone. By cotransforming such cells with the cloned human growth hormone gene and with the plasmid pdLAT-3 and then recovering Ltk⁺ aprt⁺ cells according to the method described, cells containing and expressing multiple copies of the human growth hormone gene are obtained and a method for producing human growth hormone is provided.

Another application of the invention involves introducing into eucaryotic cells multiple copies of a plasmid similar to pdLAT-3 except that a functionally deficient, promoterless, amplifiable gene coding for G418 resistance replaces the tk gene.

Still another application of the invention involves introduction of multiple copies of a cloned, carrier-free gene for human growth hormone into eucaryotic cells by cotransforming this gene and a plasmid carrying a functionally deficient, promoterless, amplifiable gene coding for G418 resistance and a functional, amplifiable aprt gene.

Experimental results

Experimental design

A system to study gene rearrangements in mammalian cells was developed based upon two predictions: 1) a gene lacking its promoter will not function when introduced into a cell in culture; and 2) rare cell mutants which do express the truncated gene will have undergone some form of genetic rearrangement.

The transcriptional control region for the structural gene encoding the Herpes virus thymidine kinase has been mapped with precision [McKnight, S. L., Gavis, E. R., Kingsbury, R. and Axel, R. (1981). Cell *25*, 385—398]. 110 nucleotides of 5' flanking DNA immediately adjacent the site of transcription initiation is essential for expression of the tk gene. Deletions extending into this region result in a striking decrease in specific transcription and a concomitant reduction in transformation efficiency. Initially, a 7.8 kb Hind III fragment containing the hamster aprt gene was inserted into a plasmid containing the intact tk gene. A unique Bgl II site is located within the 5' untranslated region of the tk gene, 50 nucleotides 5' to the initiator ATG. A second Bgl II site in the aprt-tk clone is located within the hamster DNA, 5' to all sequences necessary for aprt expression. The Bgl II fragment delineated by these two sites es excised and replaced with a 2.6 kb fragment of bacteriophage lambda DNA. The consequence of this substitution is the removal of the promoter elements and 5' cap site of the tk gene, while the coding region and poly A addition site remain intact. This plasmid contains a function aprt gene which is a selectable marker enabling us to introduce the linked, mutant tk gene into mouse Ltk⁻ aprt⁻ cells. In this plasmid, the aprt and tk genes have been introduced in opposite polarities, preventing read-through transcription of a tk transcript from an aprt promoter. Transformants constructed with this plasmid display the aprt⁺ tk⁻ phenotype. It is anticipated that tk⁺ variants hwich emerge from aprt⁺ tk⁻ transformants are the result of gross rearrangements rather than local alterations in base sequence.

Mutation to the Aprt⁺ Tk⁺ phenotype

The aprt-tk plasmid was introduced into mouse Ltk⁻, aprt⁻ cells as a calcium phosphate precipitate [Wigler, M., Sweet, R., Sim, G. K., Wold, B., Pellicer, A., Lacy, E., Maniatis, T., Silverstein, S. and Axel, R. (1979). Cell *16*, 777—785] under conditions which select for the aprt⁺ phenotype. All aprt⁺ clones remained tk⁻. Eight clones were identified which had integrated at least one copy of the aprt-tk plasmid. These characterized clones were independently isolated and tested for the frequency with which they generated tk⁺ variant subclones. Each of the eight clones was expanded in adenine-azaserine-BUdRt, growth conditions which maintain the aprt⁺ tk⁻ phenotype of the cell population. Each cell line was then growth for three generations in neutral medium (supplemented only with adenine) and then placed into growth medium that selects for the tk⁺ phenotype. All eight parental clones produced tk⁺ progeny at a unique rate ranging from $1.6\times10^{-5}$ to $1.8\times10^{-7}$ mutants per cell division. All tk⁺ progeny remained aprt⁺.

Amplification is responsible for the Tk⁺ phenotype

The structures of the aprt and tk genes in aprt⁺ tk⁻ parents as well as 44 tk⁺ mutant subclones were examined by blot hybridization. Eight tk⁻ parental clones were shown by blotting to contain one or a few integrated copies of the aprt-tk plasmid. For each clone, digestion of cellular DNA with the enzyme Pvu II produces a diagnostic 5 kb linkage fragment extending from within the aprt gene, through the spacer sequence, and includes the entire tk structural gene (see Fig. 1). A 2.0 kb fragment is also generated which is homologous only to aprt sequences. A third fragment extends from within the aprt gene into flanking DNA. A final additional band is observed which extends from DNA flanking the tk coding sequences into cellular DNA. Hybridization with probes specific for either the tk or aprt genes reveals that in all 44 tk⁺ clones the copy number of the tk and linked aprt genes has increased 20 to 40 fold. Pvu II-cut DNA was probed with a tk gene sequence and it was found that there is a single copy of the 5.0 kb linkage fragment and thus of the tk gene in the tk⁻ parent. The intensity of this fragment, as well as the 2.4 kb flanking fragment, increased dramatically in tk⁺ subclones. The extent of amplification was about 20 to 40 fold. A

third fragment, containing an incomplete tk gene, is also contained within the amplification unit. As judged by blot hybridization, concomitant amplification of the linked aprt gene was observed in all $tk^+$ mutants. The unit of DNA which has been amplified not only includes the tk-aprt plasmid, but from 40 to greater than 200 kb of flanking DNA (see below). It should be noted that in 90 percent of the clones tested, the amplification process occurred without gross rearrangement. The linkage between the aprt and tk genes was maintained intact without changes in the flanking fragments.

Amplified expression of an aberrant Tk mRNA

In order to determine the mechanism by which amplification renders the cells $tk^+$, an analysis of the RNA transcripts present in the parental $tk^-$ and amplified $tk^+$ cell lines was made. In control populations of cells transformed with a wild-type tk gene, a major 1.3 kb transcript is expressed which is responsible for the synthesis of wild-type tk enzyme. 5′ and 3′ termini of this message have been mapped with precision, utilizing an S-1 nuclease mapping assay [McKnight, S. L. and Gavis, E. R. (1980). Nucl. Acids Res. *8*, 5931—5948]. Some transformants containing a single copy of the wild-type tk gene also express a smaller 0.9 kb transcript. RNA from one such control transformant containing the two transcripts was examined by Northern blot hybridization. Cells containing a single copy of the truncated tk gene plasmid express the 0.9 kb RNA transcript. A second, less abundant 1.1 kb RNA is also expressed. Hybridization with tk probes indicates that both transcripts encode a portion of the tk structural gene. The tk transcripts from one amplified $tk^+$ mutant overproduced the 0.9 kb transcript in proportion with the level of amplification of the truncated tk gene. A second $tk^+$ mutant overproduced the 1.1 kb RNA, initially present at exceedingly low levels in the $tk^-$ parent, to a similar extent.

From these results, it is apparent that a number of alternative transcription start sites exist about the tk gene, some of which are utilized only after the primary promoter has been deleted, suggesting that the aberrantly initiated 0.9 kb message can produce sufficient tk enzyme only when present in the cytoplasm at high copy number. It is likely that this short alternative transcript encodes a similarly shortened tk enzyme, 70 percent the size of the wild-type protein. Examination of the tk gene sequence reveals three in-phase translation initiation codons close to the predicted 5′ terminus for this message. This short enzyme is likely to function poorly and only when it is present in abundance is sufficient enzymatic activity accumulated to permit conversion to the $tk^+$ phenotype.

An alternative mechanism by which amplified cell lines display the $tk^+$ phenotype involves the relative over-production of a 1.1 kb transcript. This RNA is present at very low levels in $aprt^+$ $tk^-$ cells, but its synthesis is enhanced upon amplification. These results suggest that this aberrant transcript is regulated by a weak promoter which generates sufficient mRNA only when the tk gene is present in high copy number.

Physical structure of amplified DNA

The ability to amplify transforming DNA provides a facile experimental system to study the extent and arrangement of amplified genes. Cotransformation results in the integration of concatenates of transforming DNA, consisting of a single selectable gene along with from 1 to 2,000 kb of carrier DNA at a single locus in the chromosome [Robins, D. M., Ripley, S., Henderson, A. S. and Axel, R. (1981). Cell *23*, 29—39; Perucho, M., Hanahan, D. and Wigler, M. (1980). Cell *22*, 309—317]. If the carrier DNA consists of a well-defined collection of cloned DNA sequences, the length and order of these sequences in the transforming element can be mapped with precision.

To this end, $aprt^+$ $tk^-$ cell lines were constructed with carrier DNA which consisted entirely of 20 plasmid clones containing 5—15 kb inserts of random human DNA sequences inserted into the pBR322 derivative, pAT153. Cell lines constructed in this manner contain a single copy of the tk-aprt plasmid flanked at 5—15 kb intervals by uniquely identifiable pBR sequences. $Tk^+$ mutant subclones were derived from these lines and the organization of amplified DNA was determined by examining the number and arrangement of the amplified pBR sequences.

Genomic DNA was isolated from a $tk^-$ parent and eight independently derived $tk^+$ mutant subclones, cleaved, blotted and probed for pBR sequences. The DNA was cut with Bgl II and Xba I, two enzymes which do not cut pBR, so that each integrated pBR will be displayed as a unique band upon blot hybridization. This unamplified $tk^-$ parent shows numerous pBR bands of single copy intensity. The eight $tk^+$ subclones have each amplified a subset of the pBR bands.

Four conclusions can be drawn from this experiment. First, the size of the amplification unit is variable, ranging from 40 to greater than 200 kb. By simply counting the number of amplified pBR bands, one obtains a direct estimate of the size of the amplification unit.

Second, despite a common genetic background, each amplification unit is unique. No two $tk^+$ subclones have amplified the same subset of pBR bands.

Third, despite this heterogeneity, a common core of bands is amplified in all clones, and one mutant amplified only this core. The existence of this core suggests that in all clones, amplification initiates at a common point and procedes outward, for varying distances, into flanking DNA. This common point may be the origin of replication for the amplified unit. Since the entire amplified unit consists of pBR clones which have already been identified and recovered, this origin is readily isolatable.

Finally, extensive rearrangement does not occur during amplification. In general, each amplified band

is present as a single copy band in the tk⁻ parent. In 10 percent of amplified cell lines, rearrangements about the tk and aprt genes were detected. These rearrangements coexisted with the parental gene organization, suggesting that occasionally gene variations arise during the amplification process. The variant genes are only a fraction of the amplified gene copies.

Genetic behavior of amplified DNA

The presence of two selectable markers, aprt and tk, within the amplified unit has made it possible to study the genetic behavior of the amplified DNA. It was asked whether, in a single generation, amplified clones containing multiple aprt genes can mutate to the $aprt^- tk^+$ phenotype. These phenotypic requirements are such that the tk gene must remain amplified while all the amplified aprt genes become inactivated.

Initially, $aprt^- tk^+$ mutants were isolated in the following manner. A pool of approximately 100 $aprt^+ tk^+$ colonies derived from a single unamplified $tk^-$ parent were grown in mass culture, placed in neutral medium for three generations and then placed into selection for the $aprt^- tk^+$ phenotype. $Aprt^- tk^+$ mutants derived from this population were isolated at a frequency of $1.3\times10^{-5}$. In addition, 14 independent $aprt^+ tk^+$ clones were individually analyzed for mutation rate to the $aprt^- tk^+$ phenotype. Twelve of the fourteen lines produced $aprt^- tk^+$ mutants at rates ranging from $3\times10^{-4}$ to $4\times10^{-7}$. Such rates are close to the frequencies with which cells containing a single linked wild-type aprt and wild-type tk gene mutate to the $aprt^- tk^+$ phenotype. The isolation of these mutants was therefore quite surprising. If each aprt gene were mutating independently of all other amplified aprt genes, the probability of isolating a cell containing 40 mutant aprt genes would be undetectably small.

A fluctuation analysis [Luria, S. E. and Delbruck, M. (1943). Genetics *28*, 491—511] was performed on one amplified cell line to more precisely measure the mutation rate to $aprt^-$. Table 1 summarizes the results of this experiment. The ratio of the variance to the man is approximately one in the control cultures, the value expected for a Poisson distribution. This rate is much larger in the experimental series, a result which would be expected for the random occurrence of mutations during growth in neutral medium prior to the imposition of selective agents. The mutation rate to $aprt^-$, calculated by the fraction of cultures with no mutations [Lea, D. E. and Coulson, C. A. (1949). J. Genet. *49*, 264—285] is $1.41\times10^{-4}$ mutants per cell division. Finally, this analysis demonstrates that the capacity to mutate to $aprt^-$ is a property of every cell, not a unique property of an unusual variant which may have arisen in the derivation of this cell line.

Genotypic changes in the mutation to the $aprt^-$ phenotype

The structure of the tk and aprt genes was examined by blot hybridization in seven $aprt^- tk^+$ mutants derived from a population of $aprt^+ tk^+$ clones.

TABLE 1
Fluctuation analysis*

| | Independent replicas | Mass culture |
|---|---|---|
| Number of replicate cultures | 30 | 1 |
| Number of samplings per culture | 2 | 4 |
| Initial number of cells per replicate | 20 | |
| Final number of cells per replicate | $5\times10^4$ | $5\times10^4$ |
| Number of duplicates with zero $Aprt^- Tk^+$ colonies | 1 | 0 |
| Number of $Aprt^- Tk^+$ colonies per replicate | | |
| Range | 0—3,804 | 521—563 |
| Median | 186 | 539 |
| Mean | 436.4 | 545 |
| Variance/mean | 1,604.3 | 0.49 |
| Mutation rate | | |
| $F_0$ calculation | $1.36\times10^{-4}$ | |
| Median calculation | $7.09\times10^{-4}$ | |

*For the fluctuation experiment, 30 replicate cultures of an $aprt^+$ $tk^+$ cell line were grown in confluence in neutral medium following seeding with approximately 20 cells per plate. Each plate was then reseeded in duplicate at $5\times10^4$ cells per plate and selection was imposed for the $aprt^-$ $tk^+$ phenotype. A mass culture of RA was also maintained in neutral medium and aliquots were similarly placed into selection for the $aprt^-$ $tk^+$ phenotype. Mutation rates were calculated by the methods of Lea, D. E. and Coulson, C. A. (1949) J. Genet. *49*, 264—285. The $F_0$ calculation is based upon the fraction of plates with no colonies. The median calculation is based upon the median number of $aprt^-$ $tk^+$ colonies per plate.

First, the linkage arrangement between the aprt and tk genes was determined by examining the integrity of the 5.0 kb Pvu II fragment which extends from within the aprt gene through the tk gene (see Fig. 1). Secondly, the structure of the aprt gene itself was determined from the integrity of the 4.3 kb Hind III/Bgl II fragment which contains the entire aprt gene.

This analysis revealed two classes of mutants. One class has deleted all the aprt genes. The 4.3 kb Hind III/Bgl II fragment was absent and no information homologous to aprt remained in the genome. The tk genes, as expected, remain amplified. As mentioned above, the $aprt^+$ $tk^+$ amplified clones reveal a diagnostic 5 kb Pvu II fragment reflecting the linkage between aprt and tk present in the donor plasmid. Deletion of a single aprt gene would be expected to produce a single new Pvu II linkage fragment which extends from the tk gene into flanking DNA beyond the extent of the deletion. Multiple independent deletion events required to remove all the aprt genes should therefore generate several new Pvu II fragments. It is striking that in each $aprt^-$ $tk^+$ mutant clone resulting from deletion of all aprt genes, only one amplified Pvu II fragment is observed which differs in size from the original linkage fragment. Independent $aprt^-$ $tk^+$ mutant subclones each show a different single amplified Pvu II fragment. It appears that the deletions were not independent mutation events. A single deletion event has become reiterated in all members of the amplified array.

A second, less frequent class of $aprt^-$ $tk^+$ mutants show no changes in the structure or arrangement of the tk or aprt genes. The 4.3 kb fragment containing the aprt gene along with the diagnostic Pvu II linkage fragment remain unchanged. Furthermore, the extent of amplification of the tk and aprt genes is identical in $aprt^+$ parents and the $aprt^-$ mutants.

A transformation assay was employed to determine whether the $aprt^-$ phenotype in these cell lines results from transmissible alterations in DNA or from phenotypic modulation of aprt expression without concomitant DNA changes. High molecular weight DNA was isolated from an amplified $aprt^+$ cell line and three $aprt^-$ mutants which retain the aprt gene. $Aprt^-$ cells were exposed to each of the four DNA samples and the efficiency with which each sample gave rise to $aprt^+$ colones was measured. 60 µg of DNA from each of the $aprt^-$ mutant lines produced no colonies. These data indicate that this class of mutants result from heritable changes in DNA not readily detectible by blot analyses.

Aberrant transcripts produced by mutant aprt genes

Analysis of thie RNA transcripts from these mutant genes reveals alterations in aprt mRNA consistent with the notion that in each $aprt^-$ cell line all amplified aprt genes bear an identical mutation. Cell lines containing a single copy of the wild-type hamster aprt gene express a unique 1 kb transcrip in total cellular poly $A^+$ RNA. In $aprt^+$ $tk^+$ cell lines in which the aprt gene has been coamplified with the truncated tk gene, the amount of this mRNA increases in proportion with the extent of amplification. The amplified $aprt^-$ lines, which show no changes in Southern blotting, all show changes in the pattern of RNA transcripts. One cell line was particularly instructive. It does not express the wild-type 1 kb aprt mRNA, but instead produces two aberrant transcripts, both present at amplified levels. It is suggested that these two transcripts result from a mutation which alters the wild-type pattern of splicing. A second mutant line produces two similar aberrant transcripts at strikingly reduced amounts. In a third $aprt^-$ line, a 1 kb mRNA of appropriate size is present at low level, no longer consistent with the extent of amplification. At present, the nature of these mutations or the manner in which they generate the $aprt^-$ phenotype is not understood. Nonetheless, in this class of mutants, as well as with the deletion mutants, it appears that a unique aprt mutation arose in a single aprt gene and through correction processes was transmitted to all the members of the amplified aprt array.

The physical structure of amplified DNA in $aprt^-$ mutants

In considering possible mechanisms responsible for the correction process, it is important to discern whether the sequence changes observed are restricted to a localized region about the aprt gene or whether gross reorganization extends throughout the amplified array. The physical changes in the amplification unit upon correction of cell lines constructed solely with defined plasmids were therefore examined. As described above, these cloned cell lines maintain a transforming element which consists of plasmid sequences for which cloned probes are available. In this way, a mapped amplified array can be analyzed before and after correction has occurred.

Three $aprt^+$ $tk^-$ lines containing derivatives of the plasmid pBR322 were amplified by selection for the $aprt^+$ $tk^+$ phenotype. A total of 14 amplified subclones were picked and expanded. As expected, in each of

these lines, the aprt gene remained linked to the tk gene and significant lengths of the transforming DNA have been amplified to 20 to 40 fold. Of these 14 lines, 12 produced aprt$^-$ tk$^+$ mutants at detectable frequencies. It should be emphasized that in each of these lines, the aprt and tk genes amplified faithfully. No evidence was found of the production of variant genes during the amplification process in this set of cell lines.

The structure of the aprt gene was initially examined by blot hybridization in each of these aprt$^-$ tk$^+$ cell lines. In each mutant, aprt information has been deleted. A single deletion has been reiterated in all the units comprising the amplified array. Different deletions occur in different cell lines. Furthermore, in 10 of these mutants one breakpoint of the deletion maps occurs within the aprt gene itself, demonstrating that recombination between repeating pBR sequences is not the mechanism of deletion.

pBR322 sequences were utilized as a probe to examine the organization of the flanking DNA in the amplification unit. Examination indicates that the amplification unit remains essentially unchanged upon correction. Each mutant retains the unique array of plasmid sequences displayed by its aprt$^+$ parent. Two bands amplified in the parent are absent in the aprt$^-$ mutants. Hybridization with aprt gene probe demonstrates that one of these bands contains the aprt gene deleted in the aprt$^-$ mutants. The second band may reflect one breakpoint of the deletion in flanking DNA. Thus, the correction process seems to be highly conservative. Rearrangements are restricted to a unique change about the aprt gene which appears identical in all units of the amplified array.

Chromosomal arrangement of amplified DNA

*In situ* hybridization was used to determine whether the amplified DNA in these cell lines resides within a chromosome or exists as small extrachromosomal elements such as double minute chromosomes [Kaufman, R. J., Brown, P. C. and Schimke, R. T. (1979). Proc. Nat. Acad. Sci. USA *76*, 5669—5673].

The location of the amplified DNA in one aprt$^+$ tk$^+$ cell line, RA, and a corrected aprt$^-$ tk$^+$ mutant, RA$^-$, were determined by hybridization *in situ*. These amplified tk$^+$ cell lines contain about 600 pBR sequences facilitating detection by *in situ* hybridization. Metaphase spreads were prepared from colcemid-treated cells and hybridized *in situ* with $^{125}$I-labelled pBR322.

A metaphase plate of the aprt$^+$ tk$^+$ line RA following hybridization with pBR322 was examined. Labelling was localized to a small metacentric chromosome. Examination of over 25 metaphase plates demonstrated that the amplified DNA appears to occupy a unique chromosomal region. A metaphase plate from an aprt$^-$ derivative of RA was also examined. Following hybridization, labelling was similarly localized to a small metacentric chromosome. Inspection of approximately 50 metaphase plates demonstrated that, following mutation to aprt$^-$, amplified DNA remains within the chromosome.

Discussion

Gene amplification

An aprt$^-$ tk$^-$ cell has been transformed with a plasmid containing two genes: a wild-type aprt gene linked to a tk gene lacking its promoter. Transformants which integrate a single copy of this plasmid exhibit the aprt$^+$ phenotype but remain tk$^-$. Tk$^+$ variants emerge which result from 20—50 fold amplification of the linked plasmid along with significant lengths of flanking DNA. In some cells, the mutant tk gene produces a short transcript which presumably encodes a truncated protein with reduced enzymatic activity. In other cells, aberrant transcripts result from recognition of apparently weak promoters. In both instances, multiple genes are required to generate sufficient enzyme to convert the cell to the tk$^+$ phenotype.

This experimental design selects for mutations involving gene rearrangements which activate the promoter-less tk gene. Mutations due to rearrangements such as insertion, deletion, or amplification may reflect the capacity of the cell to reorganize its genome in the performance of normal cellular processes. Rearrangement mechanisms responsible for mutation in some genes may be exploited to assure the appropriate function of other genes. Thus, deletions are essential events in the activation of immunoglobulin genes [Sakano, H., K., Heinrich, G. and Tonegawa, S. (1979). Nature *280*, 288—294]. More complex transpositions result in the expression of silent sequences determining mating type in yeast [Hicks, J. B. and Herskowitz, J. (1977). Genetics *85*, 373—393]. Inversions can both activate and inactivate subsets of surface antigens in procaryotes [Zeig, J., Hilmen, M. and Simon, M. (1978). Cell *15*, 237—244]. Finally, amplification of the chorion gene occurs in the course of larval development of Drosophila to increase the production of chorion proteins [Spradling, A. C. (1981). Cell *27*, 193—203].

Two general mechanisms have been invoked to explain the events responsible for amplification. Gradual processes, such as unequal crossing-over between sister chromatids has been suggested to explain the slow, stepwise selection required to generate amplified cell lines resistant to specific inhibitors of enzyme activity [Alt, F. W., Kellems, R. E. Bertino, J. R. and Schimke, R. T. (1978). J. Biol. Chem. *253*, 1357—1370]. Rapid amplification events may involve localized bursts of replication which escape normal control mechanisms and allow a sequence to be recognized and copied by the replicative machinery more than once per generation. In the present studies, the tk$^+$ phenotype emerges in a single step selection suggesting a rapid mechanism of DNA amplification.

The amplification of a transforming element, consisting solely of a small number of cloned sequences, permits one to characterize the physical structure of the amplified unit. Analysis of the structure of amplified endogenous genes is frequently difficult since amplification may proceed into sequences for

which cloned probes are currently unavailable. Examination of the structure of amplified DNA in several independent $tk^+$ cell lines derived from a single parent, and therefore of common genetic background, indicates that each amplification unit is unique. The size of the amplification unit is variable, ranging from 40 to greater than 200 kb. Despite this heterogeneity, a common core of bands is amplified in all clones. Finally, each amplified band is also present in single copy in the parent indicating that extensive rearrangement does not necessarily accompany the amplification process.

The observation that a common set of four core bands is amplified in all $tk^+$ lines derived from a single parent suggests that amplification may proceed from a fixed point or origin. One of these four bands should therefore contain the replicative origin and be readily isolatable. The size of the amplified unit in sibling cell lines, however, is quite variable, implying that the site of termination may not be fixed. Replication and subsequent amplification may proceed varying distances from a unique origin.

Examination of chorion gene amplification [Spradling, A. C. (1981). Cell *27*, 193—203] has demonstrated that sequences proximal to a putative chromosomal origin of replication are amplified to a greater extent than those more distal to this locus. The results reported here are consistent with this observation. Furthermore, these results suggest that such an amplification gradient may not be a property of individual cells, but rather is a property of a cell population where component cells have each uniformly amplified a more or less extensive region of chromosomal DNA.

It is likely that any selectable gene mutated *in vitro* in such a manner as to diminish but not eliminate its activity will amplify upon appropriate selection *in vivo*. Cotransformation and subsequent amplification of disabled genes, along with other clones sequences, will permit the production of large quantities of protein products encoded by the cotransformed genes. The ability to select cells which amplify cotransformed DNA also greatly simplifies the task of cloning cotransforming DNA from the recipient cell genome [Perucho, M., Hanahan, D., Lipsich, L. and Wigler, M. (1980). Nature *285*, 207—210; Lowy, I., Pellicer, A., Jackson, J., Sim, G. K. Silverstein, S. and Axel, R. (1980). Cell *22*, 817—823].

Gene correction

$Aprt^+$ $tk^+$ lines maintain an amplified array consisting of a mutant tk gene and a wild-type aprt gene. Selection for $aprt^-$ $tk^+$ variants imposes pressure to maintain the amplified tk genes but requires inactivation of over 20 wild-type aprt alleles. $Aprt^-$ $tk^+$ variants arise at inordinately high frequencies. From fluctuation analysis, a mutation rate of $10^{-4}$ can be calculated. Thus, the rate of inactivation of 20 amplified aprt genes approximates that for a single aprt gene. The results of fluctuation tests indicate that this property, the ability to mutate to the $aprt^-$ phenotype, is inherent in all cells in the population and is not restricted to a small subset of variant cells.

Selection for the $aprt^-$ phenotype is imposed in a single generation. However, the multiple genotypic events responsible for the $aprt^-$ phenotype may not occur in one generation but may accumulate in time without phenotypic consequences, until all genes have been inactivated.

Analysis of the structure of the amplified DNA as well as of the aprt mRNA indicates that within a given $aprt^-$ cell, all amplified units bear identical mutations. This suggests a two-step process in which mutation occurs in one aprt gene and is ultimately transmitted to all other aprt genes within the amplified array. This process is termed correction. Although the overall frequency of appearance of $aprt^-$ variants is measurable, one cannot directly measure the rates of its two component steps: mutation and correction. Considering the rate of mutation of a single aprt gene, the present data suggest that correction may be a highly efficient process under the experimental conditions described.

A. Contraction and re-expansion

Two basic mechanisms have been considered to explain the correction process: contraction:re-expansion and genetic recombination. In its simplest form, contraction:re-expansion would involve mutation of a single aprt gene, deletion of the remaining genes, and subsequent re-amplification to provide a sufficient number of tk genes to maintain the $tk^+$ phenotype. These events would have to occur in one or a few generations to survive the imposed selection condition. This and related explanations are considered highly unlikely. First, the pattern of amplified bands in $aprt^-$ $tk^+$ mutants is virtually identical to the $aprt^+$ $tk^+$ parent. This contrasts with the original amplification process in which all $aprt^+$ $tk^+$ derivatives of a single $aprt^+$ $tk^+$ clone reveal different amplified units. If re-expansion were occurring, one would not expect the new pattern of amplified bands to mimic the profile observed in the parent. Second, the expected frequency of individual steps in this process—mutation, deletion, re-amplification—should approximate $10^{-9}$ or less, a value five orders of magnitude lower than the observed frequency.

One form of the contraction:re-expansion model could involve the existence of amplified units on multiple extrachromosomal elements such as double minutes. Mutation could occur within one element which subsequently accumulates in one cell by random unequal segregation. This explanation is excluded in these studies by the observation that amplified DNA resides on a chromosome in both $aprt^+$ and $aprt^-$ cells.

One form of the contraction:re-expansion model consistent with the present data involves multiple cycles of replication at a single chromosomal locus without subsequent integration of the amplified DNA. This process of "local polytenization" could generate a structure resembling an onion skin [Botchan, M., Topp, W. and Sambrook, J. (1978). Cold Spring Harbor Symp. Quant. Biol. *43*, 709—719]. This process has

been suggested to explain the amplification of integrated viral genes [Botchan, M., Topp, W. and Sambrook, J. (1978). Cold Spring Harbor Symp. Quant. Biol. *43*, 709—719] as the specific developmental amplification of Drosophila chorion genes [Spradling, A. C. (1981). Cell *27*, 193—203]. Adapting this model to these studies, amplification in the $aprt^+$ $tk^-$ parent would require the presence of a mutant origin of replication in the vicinity of the integrated aprt-tk plasmid. The properties of this mutant origin would permit a localized region of DNA to escape normal growth controls and undergo multiple rounds of replication within a single cell cycle. In the next generation, all unintegrated amplified DNA must be discarded and the mutant origin then regenerates the "onion-skin" during each successive cycle. It should be noted that the involvement of mutant origins requires that the amplified DNA be discarded at each generation to prevent the exponential accumulation of amplified sequences with each successive cell cycle. In such a model, mutation from $aprt^+$ $tk^+$ to $aprt^-$ $tk^+$ need only require mutation within the single integrated aprt gene. The wild-type unintegrated aprt DNA is discarded at the next generation and the mutated, integrated DNA replicates to an amplified state. This interesting possibility is consistent with the present data and is open to experimental test.

B. Genetic recombination

One final mechanism for correction which must be considered involves genetic recombination. In eucaryotes, individual repeat units in a tandem array frequently show striking homogeneity. Maintenance of sequence homogeneity is thought to arise either by unequal crossing-over events or gene conversion [Smith, G. P. (1976). Science *191*, 528—535; Szostak, J. W. and Wu, R. (1980). Nature *284*, 426—430; Klein, H. L. and Petes, J. D. (1981). Nature *289*, 144—148]. Direct estimates of the frequency of unequal sister strand exchange among repeated DNA sequences in mitotic yeast cells suggest this mechanism can maintain the observed homogeneity [Szostak, J. W. and Wu, R. (1980). Nature *284*, 426—430]. Unequal crossing-over is considered as unlikely explanation for the fixation of the mutant aprt gene for two reasons. First, unequal crossing-over between the highly repeated pBR sequences in these cell lines would scramble their relationship to one another, resulting in an altered Southern blot banding pattern, which is not the case. Second, only 30 generations elapse between the creation of these cell lines and the imposition of selection for the $aprt^-$ phenotype. Computer simulations of the unequal crossing-over process has indicated that the probability of fixation of a mutant allele in an array of 30 wild-type genes in such a few generations less than $10^{-8}$, assuming one sister chromatid exchange per generation.

Gene conversion may occur upon strand exchange between sequences of incomplete homology. Regions of non-homology are then corrected, frequently in a non-reciprocal fashion. Recent analysis of the genetic behavior of gene duplications in yeast suggest conversion is the most frequent mechanism of recombination between duplicated elements [Klein, H. L. and Petes, J. D. (1981). Nature *289*, 144—148; Jackson, J. A. and Fink, G. E. (1981). Nature *292*, 306—311]. Correction to the $aprt^-$ state may therefore result from repeated strand pairing between a mutant and wild-type aprt gene and subsequent conversion of all wild-type genes to the mutant genotype. This mechanism must operate with high frequency to account for the correction observed.

Gene conversion has been implicated in the developmental process of mating type interconversion in yeast [Haber, J. E., Rogers, D. T. and McCusker, J. H. (1980). Cell *22*, 277—289; Klar, A. J. McIndoo, J., Strathern, J. W. and Hicks, J. B. (1980). Cell *22*, 291—298] and in the maintenance of sequence identity among repeated genes [Sleightom, J. L. Blechl, A. E. and Smithies, O. (1980). Cell *21*, 627—638; Scherer, S., and Davis, R. W. (1980). Science *209*, 1380—1384; Jackson, J. A. and Fink, G. R. (1981). Nature *292*, 306—311]. This mechanism may thus be important in maintaining other essential processes in somatic cells but is evident only when appropriate selection is imposed. Whatever the mechanism for correction of amplified aprt genes in these studies, it is apparent that the transformants analyzed possess a sophisticated mechanism designed to maintain sequence homogeneity among repeated genetic elements.

Experimental procedures

Cell culture

Murine $Ltk^-$ $aprt^-$ cells were maintained in Dulbecco's modified Eagle's medium (DME) supplemented with 10 percent calf serum (M. A. Bioproducts) and 50 μg/ml diaminopurine (DAP). $Aprt^+$ $tk^-$ transformants were selected in DME, 10 percent calf serum, 4 μg/ml azaserine, 15 μg/ml adenine (DME+AzAd) [Wigler, M., Sweet, R., Sim, G. K. Wold, B., Pellicer, A., Lacy, E., Maniatis, T., Silverstein, S. and Axel, R. (1979). Cell *16*, 777—785]. $Aprt^+$ $tk^-$ transformants were maintained in DME+AzAd plus 30 μg/ml BUdR. $Aprt^+$ $tk^+$ mutants were selected in DME, 10 percent calf serum, 15 μg/ml hypoxanthine, 1 μg/ml aminopterin, 5 μg/ml thymidine (DME+HAT) [Wigler, M., Silverstein, S., Lee, S. L. Pellicer, A., Cheng, Y. and Axel, R. (1977). Cell *11*, 223—232]. $Aprt^+$ $tk^+$ cell lines were subsequently maintained in DME, 10 percent calf serum, 15 μg/ml adenine, 1 μg/ml aminopterin, and 5 μg/ml thymidine (DME+AAT). $Aprt^-$ $tk^+$ mutants were selected and maintained in DME+HAT and 100 μg/ml diaminopurine (DME+HAT+DAP).

Transformation of $Ltk^-$ $aprt^-$ cells

$Ltk^-$ $aprt^-$ cells were transformed with 20 ng of the aprt-tk construct, plasmid pdLAT-3, (linearized with Sal I) as described previously [Graham, F. L. and van der Eb, A. J. (1973). Virology *52*, 456—467; Wigler, M., Silverstein, S., Lee, S. L., Pellicer, A., Cheng, Y. and Axel, R. (1977). Cell *11*, 223—232]. Some cell lines were

cotransformed with 1 μg of carrier DNA consisting entirely of 20 different random clones of human DNA constructed by inserting human DNA into the pBR322 derivative, pAT-153.

Selection for $Tk^+$ $Aprt^+$ mutant subclones

$Aprt^+$ $tk^-$ transformants were expanded in DME+AzAd+BUdR. Cells were then grown in neutral medium for three generations (DME+Adenine) and then split out to $5\times10^5$ cells per plate in DME+AAT. Mutation rates were calculated using the formula rate:

$$21n2(M/N)/G$$

where M=number of mutant colonies isolated; N=number of cells placed into selection and G=number of generations growth in neutral medium.

Selection for $Aprt^-$ $Tk^+$ mutants

$Aprt^+$ $tk^-$ clones were amplified by selection for the $aprt^+$ $tk^+$ phenotype. Such clones were picked and expanded for approximately 20 generations on selective medium (DME+AAT). Celsl were then replated at $5\times10^5$ cells per plate, grown in neutral medium for three days (DME+hypoxanthine+adenine+thymidine) and then placed into selection for the $aprt^-$ $tk^+$ phenotype (DME+HAT+DAP). Mutation rates were calculated as described above.

Blot hybridization

Cell DNAs were digested with 2u nuclease/μg DNA for 2 hours. Sampleswere electrophoresed on 0.8 percent agarose slab gels (10 μg DNA per slot) in 40 mM Tris, 4 mM NaAcetate, 1 mM EDTA, pH 7.9. DNA fragments were transferred to nitrocellulose paper [Southern, E. M. (1973). J. Mol. Biol. *98*, 503—517], and the filters were hybridized, washed, and exposed to X-ray film as previously described [Weinstock, R., Sweet, R., Weiss, M., Cedar, H. and Axel R. (1979). Proc. Nat. Acad. Sci. USA *75*, 1299—1303; Wigler, M., Sweet, R., Sim, G. K., Wold, B., Pellicer, A., Lacy, E., Maniatis, T., Silverstein, S. and Axel, R. (1979). Cell *16*, 777—785]. DNA probes for the tk gene [Pellicer, A., Robins, D., Wold, B., Sweet, R., Jackson, J., Lowy, I., Roberts, J. M., Sim, G. K., Silverstein, S. and Axel, R. (1980). Science *209*, 1414—1422] include either the 3.6 kb BAM HI fragment or the 1.9 kb Hinf fragment, and for the aprt gene [Lowy, I., Pellicer, A., Jackson, J., Sim, G. K. Silverstein, S. and Axel, R. (1980). Cell *22*, 817—823] the 2.0 kb Hinc II fragment. Probes were nick-translated to a specific activity of 0.5—$1\times10^9$ cpm/μg with α-$^{32}$P deoxynucleotide triphosphates [Weinstock, R., Sweet, R., Weiss, M., Cedar, H. and Axel, R. (1979). Proc. Nat. Acad. Sci. USA *75*, 1299—1303].

Total cellular RNA was prepared in a procedure employing guanidine thiocyanate and hot phenol extractions. 0.5—1.0 ml of 4 M guanidine-thiocyanate was added per 60 ml tissue culture dish. The cells are dripped into a dounce, heated to 60°C. They are then dounced until homogeneous. 10 ml of hot phenol was added to the lysate and mixed at 60° for 5 minutes. 10 ml of chloroform was added and 10 ml of NaAcetate 0.1 M, pH 5. It was shaken at 60° for 10 to 15 minutes, chilled on ice for 5 minutes and spun. The aqueous phase is removed and the extraction was repeated with phenol and chloroform. It was then shaken at 60°, chilled, spun and the extraction repeated one more time. The supernatant was ethanol precipitated.

Poly A selection was carried out as described in Aviv, H. and Leder, P. (1972) Proc. Nat. Acad. Sci. USA *69*, 1408—1412. RNA blotting onto nitrocellulose was carried out using agarose-formaldehyde gels [Lehrach, H., Diamond, D., Wozney, J. M. and Boedtker, H. (1977). Biochemistry *16*, 4743—4751] under the conditions described in Goldberg, D. (1980) Proc. Nat. Acad. Sci. USA *77*, 5794—5798.

Computer simulations

A computer program was designed to determine the probability with which a single mutant allele in an array of n wild-type alleles could comprise the entire array in m generations through sister chromatid exchange. Variables include initial number of gene copies in the repeating array, initial position in the array of the mutant allele, minimum permissible size for the array as a consequence of an unequal crossover event, and maximum extent of misregister during sister chromatid pairing.

**Claims**

1. A process for introducing into eucaryotic cells multiple copies of a foreign DNA I which comprises cotransforming suitable eucaryotic cells with said foreign DNA I and with a deoxyribonucleic acid molecule which includes a foreign DNA II and a foreign DNA III, said DNA II and DNA III carried on the same deoxyribonucleic acid molecule, said foreign DNA II including a functionally deficient, amplifiable gene coding for a first selectable or identifiable trait and whose primary promoter has been removed, said foreign DNA III including a functional amplifiable gene, which is derived from a gene coding for production of adenine phosphoribosyltransferase, said foreign DNA I including a functional gene coding for a desired proteinaceous product and said cotransformation being carried out under suitable conditions permitting selection or identification of cotransformed cells expressing the second selectable or identifiable trait encoded by the gene on DNA III but not the first selectable or identifiable trait encoded by the gene on

DNA II, recovering cotransformed eucaryotic cells expressing the second trait, cloning said cotransformed cells under appropriate conditions permitting selection or identification of cells which express the gene on DNA II and recovering said cotransformed cells expressing the gene on DNA II and thereby recovering eucaryotic cells containing multiple copies of DNA I.

2. A process according to claim 1, wherein the suitable eucaryotic cells do not express the first selectable or identifiable trait encoded by the functionally deficient, amplifiable gene carried on DNA II.

3. A process according to claim 1, wherein the suitable eucaryotic cells do not express the second selectable or identifiable trait encoded by the functional, amplifiable gene carried on DNA III.

4. A process according to claim 1, wherein the suitable eucaryotic cells do not express the first or the second trait.

5. A process according to claim 1, wherein the gene of DNA I codes for production of human or animal growth hormone, insulin, interferon, a clotting factor, an influenza virus antigen, a hepatitus virus antigen, human serum albumen or an antibody.

6. A process according to claim 1, wherein the functionally deficient, amplifiable gene carried on DNA II is derived from a gene coding for production of thymidine kinase, adenine phosphoribosyltransferase, dehydrofolate reductase or for neomycin resistance.

7. A process according to claim 1, wherein the functionally deficient, amplifiable gene carried on DNA II is a thymidine kinase gene from Herpes simplex virus whose primary promoter has been removed.

8. A process according to claim 1, wherein DNA I, DNA II and DNA III are carrier-free, cloned genes.

9. A process according to claim 1, wherein DNA I, DNA II and DNA III are carrier-free, cloned genes.

10. A process according to claim 1, wherein DNA II, DNA III and a bacterial plasmid or phage DNA are carried on the same DNA molecule.

11. A process according to claim 1, wherein said foreign DNA I is treated with calcium phosphate prior to use in cotransforming the cells.

12. A process according to claim 1, wherein DNA II and DNA III are treated with calcium phosphate prior to use in cotransforming the cells.

13. A process according to claim 1, wherein suitable eucaryotic cells are mammalian cells.

14. A process according to claim 1, wherein DNA I is incorporated into the chromosomal DNA of the cotransformed cells.

15. A process according to claim 1, wherein DNA II and DNA III are incorporated into the chromosomal DNA of the cells.

**Patentansprüche**

1. Verfahren zum Einführen von Mehrfachkopien einer Fremd-DNA I in eukaryotischen Zellen durch Cotransformation geeigneter eukaryotischer Zellen mit der Fremd-DNA I und mit einem Desoxyribonukleinsäuremolekül mit darin enthaltener Fremd-DNA II und Fremd-DNA III auf demselben Desoxyribonukleinsäuremolekül, wobei die Fremd-DNA II ein funktionell mangelhaftes, amplifizierbares Gen mit Codierung für eine erste selektierbare oder identifizierbare Eigenschaft, dessen Primärpromotor entfernt wurde, beinhaltet, die Fremd-DNA III ein funktionelles amplifizierbares Gen, das von einem Gen mit Codierung für die Produktion von Adeninphosphoribosyltransferase herrührt, umfaßt, die Fremd-DNA I ein funktionelles Gen mit Codierung für ein gewünschtes eiweißartiges Produkt enthält und die Cotransformation unter geeigneten Bedingungen, die eine Selektion oder Identifizierung cotransformierter Zellen, die zwar die durch das Gen auf der DNA III codierte zweite selektierbare oder identifizierbare Eigenschaft, jedoch nicht die durch das Gen auf der DNA II codierte erste selektierbare oder identifizierbare Eigenschaft exprimieren, gestatten, durchgeführt wird, Gewinnung cotransformierter eukaryotischer Zellen, die die zweite Eigenschaft exprimieren, Klonung der cotransformierten Zellen unter geeigneten Bedingungen, die eine Selektion oder Identifizierung von Zellen, die das Gen auf der DNA II experimieren, gestatten, und Gewinnung der betreffenden cotransformierten Zellen, die das Gen auf der DNA II exprimieren, unter gleichzeitiger Gewinnung eukaryotischer Zellen mit Mehrfachkopien der DNA I.

2. Verfahren nach Anspruch 1, dadurch gekennzeichnet, daß die geeigneten eukaryotischen Zellen die durch das funktionell mangelhafte amplifizierbare Gen auf der DNA II codierte erste selektierbare oder identifizierbare Eigenschaft nicht experimieren.

3. Verfahren nach Anspruch 1, dadurch gekennzeichnet, daß die geeigneten eukaryotischen Zellen die durch das funktionelle amplifizierbare Gen auf der DNA III codierte zweite selektierbare oder identifizierbare Eigenschaft nicht exprimieren.

4. Verfahren nach Anspruch 1, dadurch gekennzeichnet, daß die geeigneten eukaryotischen Zellen die erste oder die zweite Eigenschaft nicht exprimieren.

5. Verfahren nach Anspruch 1, dadurch gekennzeichnet, daß das Gen der DNA I eine Codierung zur Produktion von menschlichem oder tierischem Wachstumshormon, Insulin, Interferon, eines Gerinnungsfaktors, eines Influenzavirusantigens, eines Hepatitisvirusantigens, von Humanserumalbumen oder eines Antikörpers besitzt.

6. Verfahren nach Anspruch 1, dadurch gekennzeichnet, daß das funktionell mangelhafte, amplifizierbare Gen auf der DNA II von einem Gen mit Codierung für die Herstellung von Thymidinkinase, Adeninphosphoribosyltransferase, Dehydrofolatreduktase oder für Neomycinresistenz abstammt.

7. Verfahren nach Anspruch 1, dadurch gekennzeichnet, daß das funktionell mangelhafte, amplifizerbare Gen auf der DNA II aus einem Thymidinkinasegen des Herpes simplex-Virus, des Primärpromotor entfernt wurde, besteht.

8. Verfahren nach Anspruch 1, dadurch gekennzeichnet, daß es sich bei der DNA I, DNA II und DNA III um trägerfreie geklonte Gene handelt.

9. Verfahren nach Anspruch 1, dadurch gekennzeichnet, daß es sich bei der DNA I, DNA II und DNA III um trägergreie, geklonte Gene handelt.

10. Verfahren nach Anspruch 1, dadurch gekennzeichnet, daß die DNA I, die DNA III und ein bakterielles Plasmid oder eine Phagen-DNA sich auf demselben DNA-MoleküI befinden.

11. Verfahren nach Anspruch 1, dadurch gekennzeichnet, daß die Fremd-DNA I vor ihrer Benutzung zur Cotransformation der Zellen mit Calciumphosphat behandelt wird.

12. Verfahren nach Anspruch 1, dadurch gekennzeichnet, daß die DNA II und die DNA III vor ihrer Benutzung bei der Cotransformation der Zellen mit Calciumphosphat behandelt werden.

13. Verfahren nach Anspruch 1, dadurch gekennzeichnet, daß es sich bei den geeigneten eukaryotischen Zellen um Säugetierzellen handelt.

14. Verfahren nach Anspruch 1, dadurch gekennzeichnet, daß die DNA I in die Chromosomen-DNA der cotransformierten Zellen eingebaut ist.

15. Verfahren nach Anspruch 1, dadurch gekennzeichnet, daß die DNA II und die DNA III in die Chromosomen-DNA der Zellen eingebaut sind.

**Revendications**

1. Procédé d'introduction dans des cellules eucaryotes de copies multiples d'un ADN I étranger, qui comporte la cotransformation de cellules eucaryotes appropriées à l'aide dudit ADN I étranger et d'une molécule d'acide déoxyribonucléique qui comporte un ADN II étranger et un ADN III étranger, lesdits ADN II et ADN III étant portés sur la même molécule d'acide déoxyribonucléique, ledit ADN II étranger comportant un gène amplifiable fonctionnellement déficient codant pour un premier trait sélectionnable ou identifiable et dont le promoteur primaire a été enlevé, ledit ADN III étranger comportant une gène amplifiable fonctionnel qui est dérivé d'un gène codant pour la production de phosphoribosyltransférase d'adénine, ledit ADN I étranger comportant un gène fonctionnel codant pour un produit protéinique désiré et ladite cotransformation étant effectuée dans des conditions appropriées permettant la sélection ou l'identification de cellules cotransformées exprimant le second trait sélectionnable ou identifiable codé par le gène sur l'ADN III mais pas le premier trait sélectionnable ou identifiable codé par le gène sur l'ADN II, la récupération des cellules eucaryotes cotransformées exprimant le second trait, le clonage desdites cellules cotransformées dans des conditions appropriées permettant la sélection ou l'identification des cellules qui expriment le gène sur l'ADN II, et la récupération desdites cellules cotransformées exprimant le gène sur l'ADN II, pour récupérer ainsi les cellules eucaryotes contenant les copies multiples de l'ADN I.

2. Un procédé selon la revendication 1, dans lequel les cellules eucaryotes appropriées n'expriment pas le premier trait sélectionnable ou identifiable codé par le gène amplifiable fonctionnellement déficient porté par l'ADN II.

3. Un procédé selon la revendication 1, dans lequel les cellules eucaryotes appropriées n'expriment pas le second trait sélectionnable ou identifiable codé par le gène amplifiable fonctionnel porté par l'ADN III.

4. Un procédé selon la revendication 1, dans lequel les cellules eucaryotes appropriées n'expriment pas le premier ou le second trait.

5. Un procédé selon la revendication 1, dans lequel le gène d'ADN I fait un codage pour la production d'une hormone de croissance humaine ou animale, d'insuline, d'interféron, d'un facteur de coagulation, d'un antigène du virus de la grippe, d'un antigène du virus de l'hépatite, d'albumine de sérum humain ou d'un anticorps.

6. Un procédé selon la revendication 1, dans lequel le gène amplifiable fonctionnellement déficient porté par l'ADN II est dérivé d'un gène codant pour la production de kinase de thymidine, de phosphoribosyltransférase d'adénine, de réductase de déhydrofolate ou pour la résistance à la néomycine.

7. Un procédé selon la revendication 1, dans lequel le gène amplifiable fonctionnellement déficient porté par l'ADN II est un gène de kinase de thymidine provenant du virus simplex de l'herpès dont le promoteur primaire a été enlevé.

8. Un procédé selon la revendication 1, dans lequel l'ADN I, l'ADN II et l'ADN III sont des gènes clonés sans porteur.

9. Un procédé selon la revendication 1, dans lequel l'ADN I, l'ADN II et l'ADN III sont des gènes clonés sans porteur.

10. Un procédé selon la revendication 1, dans lequel l'ADN II, l'ADN III et un ADN bactérien plasmide ou phage sont portés par la même molécule d'ADN.

11. Un procédé selon la revendication 1, dans lequel ledit ADN I étranger est traité par du phosphate de calcium avant son utilisation dans la cotransformation des cellules.

12. Un procédé selon la revendication 1, dans lequel l'ADN II et l'ADN III sont traités par du phosphate de calcium avant leur utilisation dans la cotransformation des cellules.

13. Un procédé selon la revendication 1, dans lequel les cellules eucaryotes appropriées sont des cellules de mammifère.

14. Un procédé selon la revendication 1, dans lequel l'ADN I est incorporé dans l'ADN chromosomique des cellules cotransformées.

15. Un procédé selon la revendication 1, dans lequel l'ADN II et l'ADN III sont incorporés dans l'ADN chromosomique des cellules.

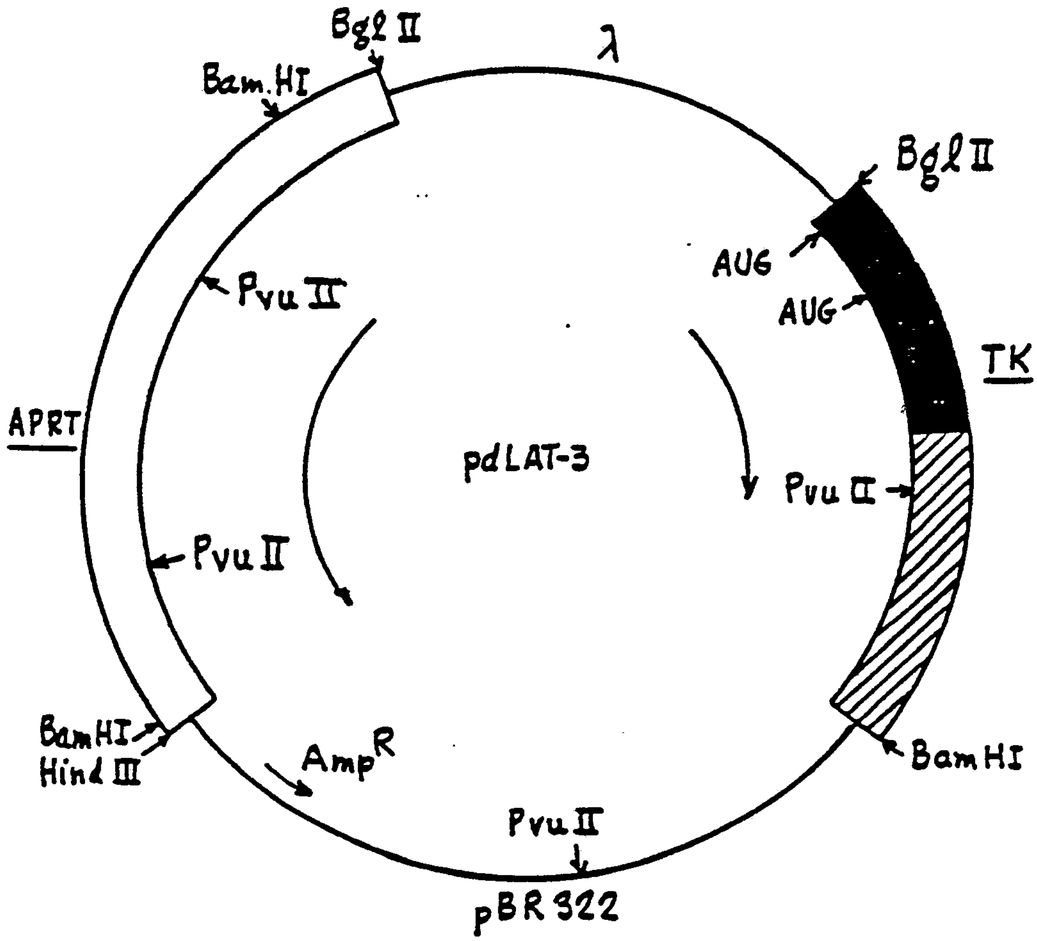
Bgl II
λ
Bam HI
Bgl II
AUG
AUG
Pvu II
TK
APRT
pd LAT-3
Pvu II
Pvu II
BamHI
Hind III
Amp^R
BamHI
Pvu II
pBR 322

Fig. 1.